(19) **Europäisches Patentamt / European Patent Office / Office européen des brevets**

(11) **EP 1 637 062 A1**

(12) **EUROPEAN PATENT APPLICATION**
published in accordance with Art. 158(3) EPC

(43) Date of publication:
**22.03.2006 Bulletin 2006/12**

(51) Int Cl.:
***A61B 1/00*** (1968.09)

(21) Application number: **04746387.2**

(86) International application number:
**PCT/JP2004/008918**

(22) Date of filing: **18.06.2004**

(87) International publication number:
**WO 2004/112591 (29.12.2004 Gazette 2004/53)**

(84) Designated Contracting States:
**DE FR GB**

(30) Priority: **19.06.2003 JP 2003175427**

(71) Applicant: **Olympus Corporation**
**Tokyo 151-0072 (JP)**

(72) Inventors:
• **Takahashi, Yoshinori,**
**c/o Olympus Corporation**
**Tokyo 151-0072 (JP)**
• **HIRAO, Isami**
 **(JP)**
• **IMAIZUMI, Katsuichi**
 **(JP)**
• **OZAWA, Takeshi**
 **(JP)**
• **TAKEHANA, Sakae**
 **(JP)**
• **DOGUCHI, Nobuyuki**
 **(JP)**

(74) Representative: **von Hellfeld, Axel**
**Wuesthoff & Wuesthoff**
**Patent- und Rechtsanwälte**
**Schweigerstrasse 2**
**81541 München (DE)**

(54) **ENDOSCOPIC DEVICE**

(57)    An endoscope device according to the present invention includes an endoscope having an image pickup device for capturing an image of a subject, which can observe the subject in a predetermined observing mode, and a signal processing device having an identifying unit that identifies the observing mode of a connected endoscope based on information from the connected endoscope, which receives a signal from the image pickup device, can execute signal processing corresponding to a plurality of observing modes including the predetermined observing mode, and further can execute only signal processing corresponding to the observing mode identified by the identifying unit.

**FIG.1**

EP 1 637 062 A1

**Description**

Technical Field

The present invention relates to an endoscope device, and more particularly, to an endoscope device that switches and executes a plurality of operating modes (observing forms) by a user.

Background Art

Recently, an endoscope device for obtaining an endoscope image of the body cavity by irradiating illuminating light is widely used. In the above-mentioned endoscope device, illuminating light from a light source is guided into the body cavity by using a light guide or the like, a subject is irradiated with the light, the return light is captured by an endoscope using a solid-state image pickup device, and the captured light is processed by a signal processing device (hereinafter, referred to as a processor), thereby displaying an endoscope image on an observing monitor and obtaining the tissue of the living body.

In the case of normally observing the tissue of the living body with the endoscope device, a light source emits white light (hereinafter, referred to as normal light) within the range of visible light, the subject is irradiated with field-sequential light via rotary filters for R, G, and B, and an image signal based on the return light is synchronized by a processor and is subjected to image processing, thereby obtaining a color image. Or, color chips are arranged on the front surface of an image pickup surface of the solid-state image pickup device of the endoscope, the return light using the normal light is split into R, G, and B colors by the color chips, and the color signals are subjected to image processing by the processor, thereby obtaining a color image.

On the other hand, since the light absorbing property and the light scattering property are varied depending on the wavelength of the light irradiated in the tissue of the body cavity, various endoscope devices for observation with specific light are proposed. For example, as recently disclosed in Japanese Unexamined Patent Application Publication No. 2002-336196, an endoscope device for observation with fluorescent light is proposed to irradiate ultraviolet light or blue light as excitation light to the tissue of the living body by using the variation in self-fluorescence generated from the tissue of the living body depending on the normal portion and the lesion for diagnosis. Further, Japanese Unexamined Patent Application Publication No. 2000-41942 proposes an endoscope device for observation with infrared light, in which infrared light is irradiated, as illuminating light, to the tissue of the living body, and the deep portion of the tissue of the living body is observed. Further, Japanese Unexamined Patent Application Publication No. 2002-95635 proposes an endoscope device for observation with narrow-band light, in which blue light with a narrow band is irradiated, as illuminating light, to the tissue of the living body, and a surface layer of the mucous membrane in the tissue of the living body can be observed.

The endoscopes used for the observation can be used for at least two types of observation including one type of observation with normal light and at least one type of observation with specific light. For example, an endoscope for observation with fluorescent light can execute observation with normal light and observation with fluorescent light. The endoscope device for observation with infrared light can execute observation with normal light and observation with infrared light. The endoscope device for observation with narrow-band light can execute observation with normal light and observation with narrow-band light.

Then, the switching operation of the observation with normal light and the observation with specific light in the endoscope device for observation with specific light is performed by key operation of a switch or a keyboard arranged to an operating portion or a processor in the endoscope or a front panel of the light source.

However, recently, a plurality of observing modes with specific light is strongly desired for use in a single processor or a single light-source. For example, one person uses an endoscope as an endoscope for fluorescent light and another person uses an endoscope as an endoscope for observing infrared light, that is, the endoscope is variously used depending on the user. In the observation with normal light, the endoscope is switched to the endoscope with observation with fluorescent light so as to precisely specify the position of the tissue, after specifying the position, the endoscope is switched to the endoscope for observation with narrow-band light so as to specifically observe the tissue near the surface of mucous membrane or blood vessel, that is, a plurality of observing modes with specific light are used for one-time examination.

However, in the observation with specific light, depending on the observing modes, the spectroscopy property of illuminating light from the light source, the transmitting property of an objective optical system of the endoscope, the type of the solid-state image pickup device, and signal processing in the processor device are varied. Thereamong, the light source and the processor can be easily designed corresponding to all observing modes. On the other hand, since the endoscope is inserted in the living body, the allowable diameter of the endoscope is limited, an objective optical system and an image pickup device corresponding to all observing modes are not included in one endoscope.

Therefore, the endoscopes need to have varying specifications depending on the observing modes. For example, the endoscope for observation with fluorescent light corresponds to three observing modes including the observation with normal light, the observation with fluorescent light, and the observation with narrow-band light. The endoscope for observing infrared light corresponds to three observing modes including the observation with normal light, the observation with infrared light, and the observation with narrow-band light. The endoscope for observation with normal light corresponds to two observing modes including the observation with normal light and the observation with narrow-band light. Incidentally, the endoscope for observation with normal light is used for the observation with narrow-band light, and therefore corresponds to any endoscope.

However, the light source and the processor correspond to all observing modes. Therefore, in the switching operation of the sequential observing mode with a single switch, an observing mode to which the connected endoscope does not correspond is possibly selected. Further, unnecessary switch operation is required for the user and it has a problem that the switching operation of the observation with normal light and the observation with specific light is troublesome and the efficiency for examination deteriorates.

The individual endoscopes have the priority of the corresponding observing mode with specific light. For example, the endoscope for observation with fluorescent light corresponds to two observations with specific light including the observation with fluorescent light and the observation with narrow-band light. The observation with narrow-band light is an observing mode which is used by another endoscope and the observation with fluorescent light has the higher priority of the observation with fluorescent light among the observing modes with specific light.

However, the order of high priority in the light source and the processor is set to (observation with normal light → observation with narrow-band light → observation with infrared light → observation with fluorescent light). Then, there is a problem that although the endoscope for observation with fluorescent light is connected, the observation with narrow-band light with low priority is first selected and the priority of the observing mode corresponding to the type of endoscope is not reflected.

Then, the present invention is devised in consideration of the above circumstances, and it is an object of the present invention to provide an endoscope device corresponding to a plurality of observing modes, in which only the observing mode to which the connected endoscope corresponds is selected by switching the observing modes.

Disclosure of Invention

An endoscope device according to the present invention includes an endoscope having an image pickup device for capturing an image of a subject, which can observe the subject in a predetermined observing mode, and a signal processing device having an identifying unit that identifies the observing mode of a connected endoscope based on information from the connected endoscope, which receives a signal from the image pickup device, can execute signal processing corresponding to a plurality of observing modes including the predetermined observing mode, and further can execute only signal processing corresponding to the observing mode identified by the identifying unit.

Brief Description of the Drawings

Fig. 1 is a block diagram showing the entire structure of an endoscope device according to a first embodiment of the present invention;
Fig. 2 is a diagram showing a filter turret plate in the endoscope device according to the first embodiment;
Fig. 3 is a diagram showing a rotary filter plate in the endoscope device according to the first embodiment;
Fig. 4 is a graph showing the transmitting property of R, G, and B filters in the endoscope device according to the first embodiment;
Fig. 5 is a graph showing the transmitting property of a filter for observation with fluorescent light in the endoscope device according to the first embodiment;
Fig. 6 is a block diagram showing the structure of a CCD with high sensitivity in the endoscope device according to the first embodiment;
Fig. 7 is a block diagram showing a color balance correcting circuit in the endoscope device according to the first embodiment;
Fig. 8 is a block diagram showing the structure of a structure emphasizing circuit in the endoscope device according to the first embodiment;
Fig. 9 is an explanatory diagram of the principle of an electronic shutter in the endoscope device according to the first embodiment;
Fig. 10 is an explanatory diagram of the electronic shutter having speeds varied depending on colors in the endoscope device according to the first embodiment;
Fig. 11 is a timing chart showing the correction in variation of color balances in the adjustment of the duty ratio of lamp driving current in the endoscope device according to the first embodiment;
Fig. 12 is a block diagram showing the entire structure of an endoscope device according to a second embodiment of the present invention;
Fig. 13 is a block diagram showing the entire structure of an endoscope device according to a third embodiment of the present invention; and
Fig. 14 is a front view showing the structure of a keyboard in the endoscope device according to the third embodiment.

Best Mode for Carrying Out the Invention

Embodiments of the present invention will be described with reference to the drawings.

[First embodiment]

It is an object of a first embodiment of the present invention to provide an endoscope device corresponding to the observation with normal light and at least one observation with specific light, in which the observing mode to which the connected endoscope corresponds is switched to the observing mode with higher priority and an image is properly observed corresponding to the observing mode in accordance with the switching operation.

[Structure]

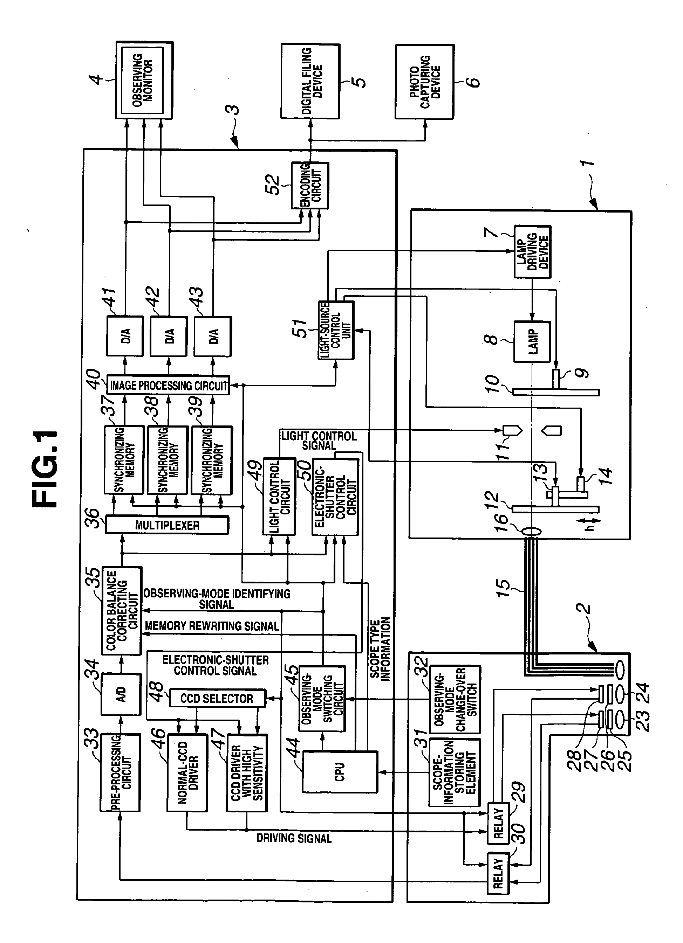

Fig. 1 is a block diagram showing the entire structure of an endoscope device according to the first embodiment of the present invention.

Referring to Fig. 1, the endoscope device according to the first embodiment comprises: a light source 1 that generates light for observation; an endoscope (hereinafter, referred to as a scope) 2 that is inserted in the body cavity; a processor 3 that processes an image signal that is captured by the scope 2; an observing monitor 4 that displays an endoscope image; a digital filing device 5 that records an encoded endoscope image as a compressed image; and a photographing device 6 that records the endoscope image as a picture.

The light source 1 comprises: a lamp driving circuit 7 that drives a lamp; a lamp 8, such as a xenon lamp, which irradiates the light; a filter turret 10 that is arranged onto the illuminating light path of the lamp 8 and switches a plurality of optical filters with transmitting wavelength bands varied depending on the observing modes by driving a motor 9; an illuminating-light stop 11 that limits the amount of illuminating light; a rotary filter 12 that coverts the illuminating light to R, G, and B field-sequential light; a motor 13 that rotates the rotary filter 12; a motor 14 that moves the rotary filter 12 in a vertical direction h of the optical axis so as to use the filters arranged on the inner circumference and the outer circumference of the rotary filter 12; and a condenser lens 16 that condenses the field-sequential light to an incident surface of a light guide 15 of the scope 2 via the rotary filter 12.

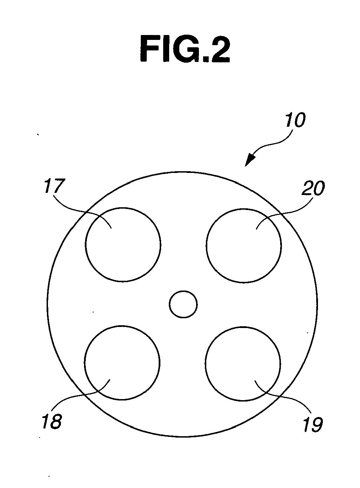

Referring to Fig. 2, the filter turret 10 is disc-shaped and comprises a plurality of optical filters with transmitting wavelength bands varied depending on the observing modes with the rotating axis as center. In the filter turret 10, the optical filter corresponding to the selected observing mode is fixed on the optical path. According to the first embodiment, the filter turret 10 comprises, as the optical filters, a filter 17 for observation with normal light, a filter 18 for observation with fluorescent light, a filter 19 for observation with infrared light, and a filter 20 for observation with narrow-band light.

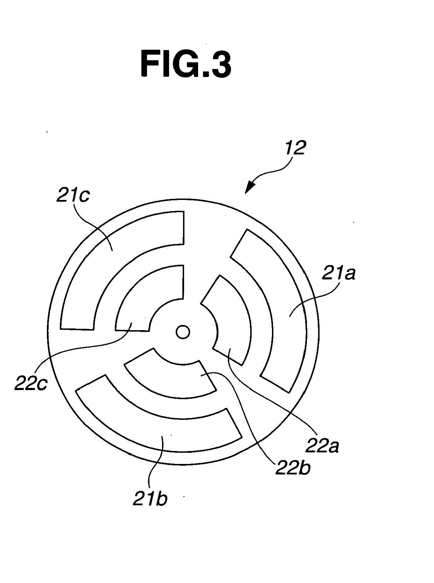

Referring to Fig. 3, the rotary filter 12 is disc-shaped, has double structure with the rotating axis as center, and comprises, on the outer circumference, R filter 21a, G filter 21b, and B filter 21c through which light with red, green, and blue wavelengths pass. The rotary filter 12 comprises, on the inner circumference, a G' filter 22a through which light with narrow band of 540 to 560 nm passes, an exciting filter 22b through which excitation light having wavelengths of 395 to 475 nm passes, and an R' filter 22c through which light with a narrow band of 600 to 620 nm passes. The portion except for the arrangement portions of the filter in the rotary filter 12 comprises a light shading member.

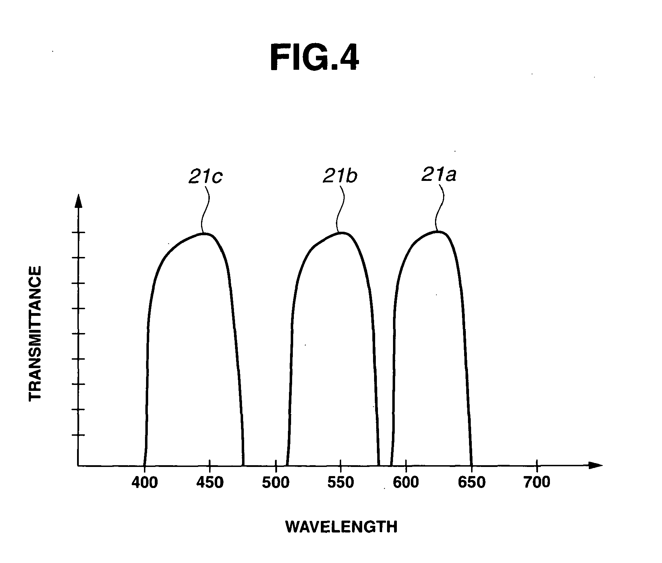

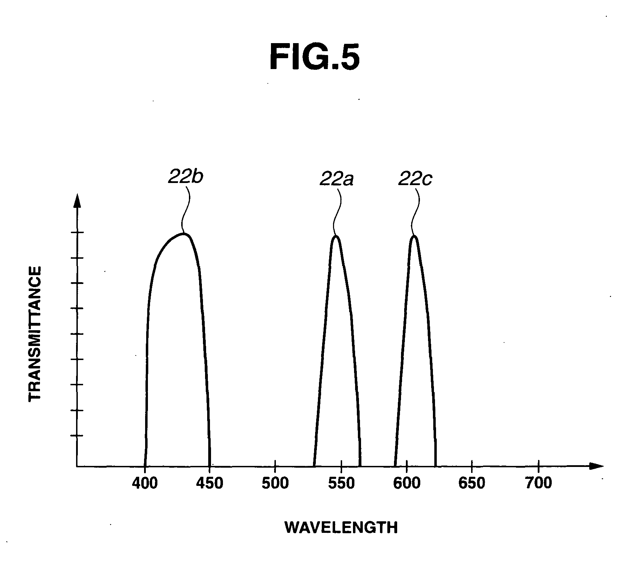

The spectroscopy properties of inner-circumferential and outer-circumferential filters are as shown in Figs. 4 and 5. Fig. 4 is a graph showing the transmitting properties of the R, G, and B filters. Fig. 5 is a graph showing the transmitting properties of the filter for observation with fluorescent light, with the abscissa as a wavelength and the ordinate as a transmittance.

As a combining result of the filter turret 10 and the rotary filter 12, the illuminating wavelength in the observing mode with specific light includes exciting wavelengths of 395 to 475 nm or 395 to 445 nm in the observing mode with fluorescent light, wavelengths of 540 to 560 nm of green reflected light, wavelengths of 600 to 620 nm of red reflected light, three wavelengths of 940 nm, 805 nm, and 805 nm as center wavelengths in the observing mode with infrared light, and three wavelengths of 415 nm, 540 nm, and 610 nm, as the central wavelength in the observing mode with narrow-band light. Incidentally, in the observing mode with normal light, although via the filter 17 for observation with normal light of the filter turret 10, the visible light passes through the filter 17 and the spectroscopy property is the same as that shown in Fig. 4.

The scope 2 comprises: the light guide 15 that transmits illuminating light incident from the light source 1 to the distal end of the scope; objective lenses 23 and 24 that receive the return light from the subject based on the illuminating light; optical filters 25 and 26; a normal CCD 27 used as an image pickup device and a CCD 28 with high sensitivity for observation with fluorescent light; relay switches 29 and 30 that comprises a plurality of switching elements and switches a driving signal of the CCD 27 or CCD 28 with high sensitivity and an image signal (CCD output signal) after image pickup operation; a scope-information storing element 31 that stores information on the observing mode corresponding to the scope 2, the priority of the observing mode, and the speed of electronic shutter; and an observing-mode change-over switch 32 that switches the observing mode by the operation of the switch.

In the processor 3, an image signal sequentially flows in the order of a pre-processing circuit 33, an A/D converting circuit 34, a color balance correcting circuit 35, a multiplexer 36, synchronizing memories 37, 38, and 39, an image processing circuit 40, and D/A converting circuits 41, 42, and 43. Further, the processor 3 comprises a CPU 44, an observing-mode switching circuit 45, a normal CCD driver 46, a CCD driver 47 with high sensitivity, a CCD selector 48, a light control circuit 49, an electronic-shutter control circuit 50, a light-source control circuit 51, and an encoding circuit 52.

[Operation]

When the scope 2 is connected to the light source 1 and the processing 3 and then the power is turned on, the endoscope device starts in the observing mode with normal light. Simultaneously to the start operation, a scope-information storing element 31 in the scope 2 reads the type of observing mode corresponding to the scope 2 and its priority to the CPU 44 in the processor 3 and stores the read information.

On the other hand, the scope-information storing element 31 does not store the observing mode without corresponding to the scope 2. Therefore, the observing mode after the start operation is switched based on the information stored in the CPU 44. In the switching operation of the observing mode with only the switch 32, the observing mode with higher priority is sequentially switched by each switching operation. When all observing modes are switched, the mode returns to the observation with normal light.

Upon switching the observing mode, by pressing the observing-mode change-over switch 32 arranged to the operating unit in the scope 2, a signal for instructing the switching operation of the observing mode is generated, and the generated signal is inputted to the observing-mode switching circuit 45 in the processor 3. Simultaneously, the observing mode of the scope 2 stored in the CPU 44 and the priority are stored in the observing-mode switching circuit 45. The observing-mode switching circuit 45 receives a signal from the observing-mode change-over switch 32 and outputs a observing-mode identifying signal indicating the observing mode after the switching operation based on information on the observing mode which is operated just before pressing the observing-mode change-over switch 32 and information on the priority of the just-before observing mode. The information on the priority of the new observing mode is stored in a memory (not shown) arranged in the observing-mode switching circuit 45.

The observing-mode identifying signal outputted from the observing-mode switching circuit 45 is transmitted to the CCD selector 48, the color balance correcting circuit 35, the synchronizing memories 37, 38, and 39, the image processing circuit 40, the light control circuit 49, the electronic-shutter control circuit 50, and the light-source control circuit 51 in the processor 3, and the relay switches 29 and 30 in the scope 2.

The CCD selector 48 determines, based on the observing-mode identifying signal, whether or not the observing mode after the switching operation is the observation with fluorescent light. When it is determined that the observing mode is the observation with fluorescent light, the self-fluorescence from the tissue of the living body irradiated with excitation light is observed. However, the self-fluorescence has excessively low light and therefore the CCD 28 with high sensitivity is used in many cases.

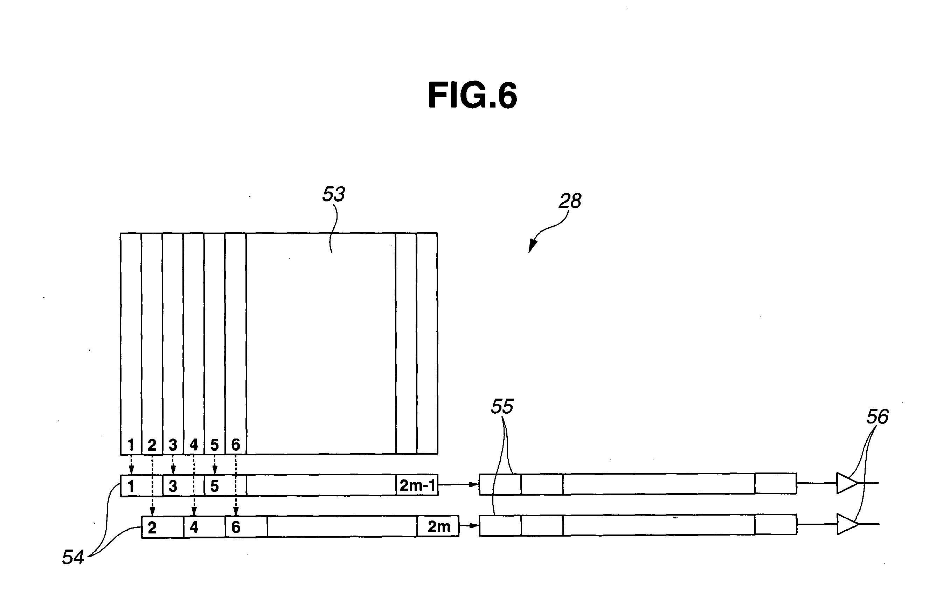

As disclosed in U.S. Patent Publication No. 5,337,340, a CCD is used, as the CCD 28 with high sensitivity, to receive a control pulse from the outside thereof, thereby controlling the amplification ratio of signals therein. Fig. 6 is an explanatory diagram of the CCD with high sensitivity.

Referring to Fig. 6, in the CCD with high sensitivity, a CMD (Charge Multiplication Device) arranged therein can increase the charges using the ionization. The CMDs can be arranged to pixels, thereby amplifying the signals for the individual pixels. Or, the CMDs may be arranged to a transfer channel, thereby amplifying the signals for individual lines.

Recently, a CCD for controlling the CMD by a voltage value is proposed without using the control pulse. Since the CCD using the CMD amplifies the signal before reading the charges, the influence from noises is suppressed, as compared with the amplification outside the CCD. There is a merit that an image with a high S/N ratio is obtained. Therefore, the image pickup operation is possible with high sensitivity and is suitable to the image pickup operation with the low light, such as fluorescent light.

Referring to Fig. 6, in a light receiving area in which a large number of light receiving elements (not shown) are arranged in the vertical and horizontal directions like matrix, a plurality of pixel trains in the vertical direction are divided into pixel trains having an odd-numbered one and an even-numbered one, the pixels are transferred to two horizontal transfer channels 54 from the alternating pixel trains of the odd-numbered one and the even-numbered one. Further, the pixels are transmitted via transfer channels 55 with the CMD which s serially connected to the horizontal transfer channels 54 and charge detecting units 56 detect the signal charge.

On the other hand, in the observing modes (observation with normal light, observation with infrared, and observation with narrow-band light) excluding the observation with fluorescent light, the normal CCD 27 is used. When the CCD selector 48 determines that the observing mode after the switching operation is the observation with fluorescent light, the CCD selector 48 outputs a signal for instructing the stop of generation of the driving signal of the CCD 27 to the normal CCD driver 46 and simultaneously outputs a signal for instructing the generation of the driving signal of the CCD 28 with high sensitivity to the CCD driver 47 with high sensitivity.

On the contrary, the current observing mode is the observation with fluorescent light and the observing mode after the switching operation is another mode, the CCD selector 48 then outputs a signal for instructing the stop of generation of the driving signal of the CCD 28 with high sensitivity to the CCD driver 47 with high sensitivity and simultaneously outputs a signal for instructing the generation of the driving signal of the CCD 27 to the normal CCD driver 46. In the switching operation between the observing modes other than the observation with fluorescent light, the CCD 27 is driven yet and therefore the CCD selector 48 does not output any signals.

The CCD driving signal outputted from the normal CCD driver 46 or the CCD driver 47 with high sensitivity is inputted to the relay switch 29 in the scope 2. The relay switch 29 is switched based on the observing-mode identifying signal outputted from the observing-mode switching circuit 45 of the processor 3. In the observation with fluorescent light, the driving signal outputted from the CCD driver 47 with high sensitivity is outputted to the CCD 28 with high sensitivity. On the other hand, in the observing mode except for the observation with fluorescent light, the driving signal outputted from the normal CCD driver 46 is outputted to the CCD 27.

Thus, any of the CCD 27 and the CCD 28 with high sensitivity is driven. An image signal (CCD output signal) of a subject captured by the CCD 27 or the CCD 28 with high sensitivity is inputted to the processor 3 via the relay switch 30. Incidentally, the relay switches 29 and 30 may be mechanical style or electronic style.

The image signal inputted to the processor 3 is first inputted to the pre-processing circuit 33. The pre-processing circuit 33 extracts the image signal by processing including CDS (correlation double-sampling). The signal outputted from the pre-processing circuit 33 is A/D converted by the A/D converting circuit 34 and is then inputted to the color balance correcting circuit 35. The circuit is called a white balance circuit in the observation with normal light.

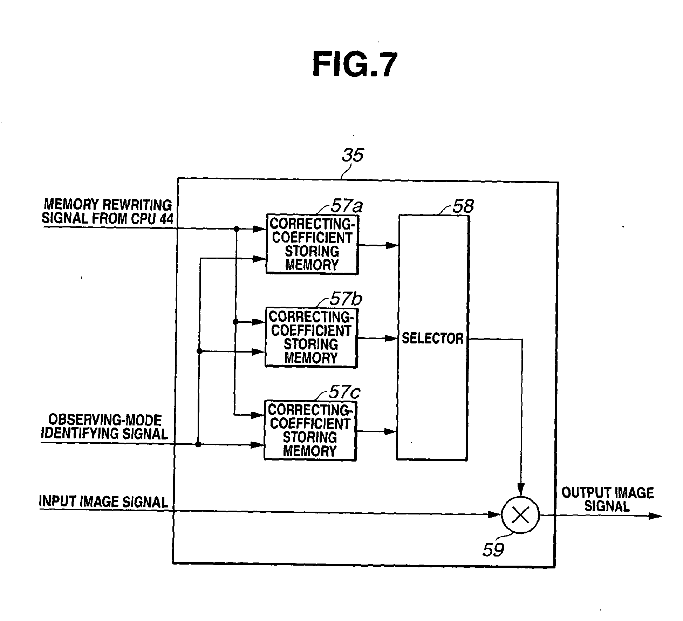

Referring to Fig. 7, the color balance correcting circuit 35 comprises: color balance correcting-coefficient storing memories 57a, 57b, and 57c, serving as non-volatile memories, which store three color balance correcting coefficients; a selector 58 which selects the color balance correcting coefficient; and a multiplier 59.

The selector 58 selects the color balance correcting-coefficient storing memory 57a at the timing for inserting the R filter 21a or G' filter 22a in the optical path, further selects the color balance correcting-coefficient storing memory 57b at the timing for inserting the G filter 21b or the exciting filter 22b in the optical path, and further selects the color balance correcting-coefficient storing memory 57c at the timing for inserting the B filter 21c or the R' filter 22c in the optical path.

The multiplier 59 multiplies the inputted image signal and the color balance correcting coefficient selected by the selector 58, and outputs the multiplied signal. To the color balance correcting-coefficient storing memories, the color balance correcting coefficients calculated by the CPU 44 are written. the color balance correcting-coefficient storing memories 57a, 57b, and 57c stores and reads the color balance correcting coefficient by the observing-mode identifying signal inputted from the observing-mode switching circuit 45 to address areas varied depending on the observing modes.

The image signal outputted from the color balance correcting circuit 35 is synchronized for field-sequential light by the multiplexer 36 and the synchronizing memories 37, 38, and 39, and is inputted to the image processing circuit 40. The image processing circuit 40 performs gamma correction, structure emphasis, and color processing. The image processing is properly performed in accordance with the observing mode by the observing-mode identifying signal from the observing-mode switching circuit 45.

For example, in the structure emphasis, a highfrequency component of the image is emphasized, using a spatial filter, such as an edge emphasizing filter or an edge emphasizing filter. The degree of structure emphasis necessary for diagnosis varies depending on the observation of the fine structure, such as the tissue of living body, in the observation with normal light and the observation with narrow-band light and the diagnosis as whether or not the affected part exists, in the observation with fluorescent light.

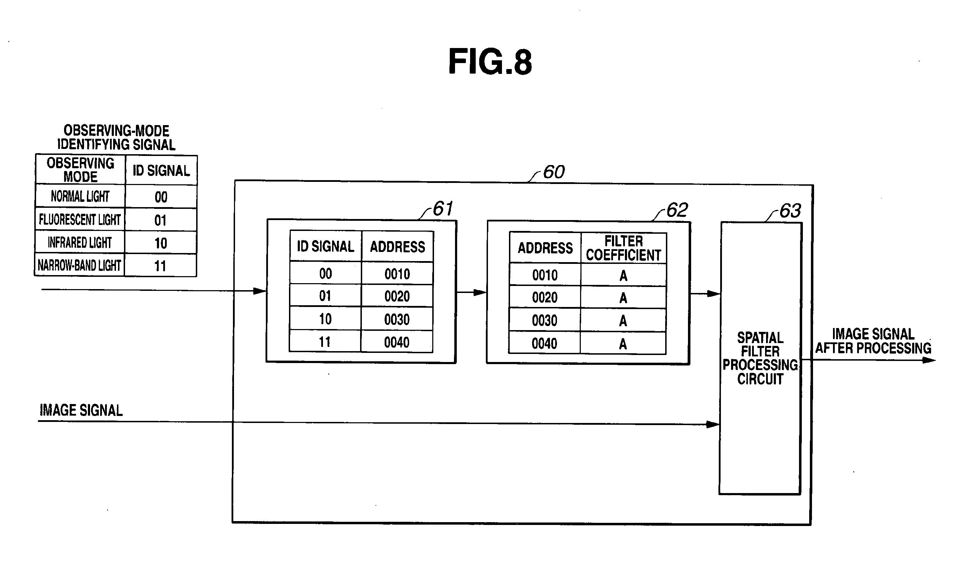

Referring to Fig. 8, the observing-mode identifying signal inputted to a structure emphasizing circuit 60 in the image processing circuit 40 is converted into an address value varied depending on the observing mode by an address generating circuit 61. The converted signal is inputted to a filter-coefficient storing memory 62. The filter-coefficient storing memory 62 has the number of combination of the address value and the filter coefficient stored in the area of the address value corresponding to the number of observing modes. In accordance with the address value inputted from the address generating circuit 61, a proper filter coefficient is outputted to a spatial filter processing circuit 63, thereby outputting the image signal which is subjected to the structure emphasis corresponding to the observing mode.

The image signal outputted from the image processing circuit 40 is converted into an analog signal again by the D/A converting circuits 41, 42, and 43, the analog signals are displayed on the observing monitor 4, and the outputs from the D/A converting circuits 41, 42, and 43 are encoded by the encoding circuit 44, thereby recording the encoded signal to the digital filing device 5 or the photographing device 6.

The light control circuit 49 outputs a light control signal for adjusting an illuminating-light stop 11 of the light source 1 based on the image signal outputted from the color balance correcting circuit 35 and the observing-mode identifying signal outputted from the observing-mode switching circuit 45 so that the image has a proper brightness in the selected observing mode. In the shortage of amount of light, the light control signal operates the illuminating-light stop 11 in the releasing direction thereof. On the contrary, when the amount of light is excessive, the illuminating-light stop 11 operates in the closed direction thereof.

The electronic-shutter control circuit 50 has a speed storing memory (not shown) of electronic shutter for storing the speed of electronic shutter in all observing modes compatible with the scope 2, which is outputted from the scope-information storing element 31. With the observing-mode identifying signal outputted from the observing-mode switching circuit 45, the proper speed of electronic shutter is read from a predetermined position of the speed storing memory of electronic shutter, and a pulse for controlling the electronic shutter is generated and outputted.

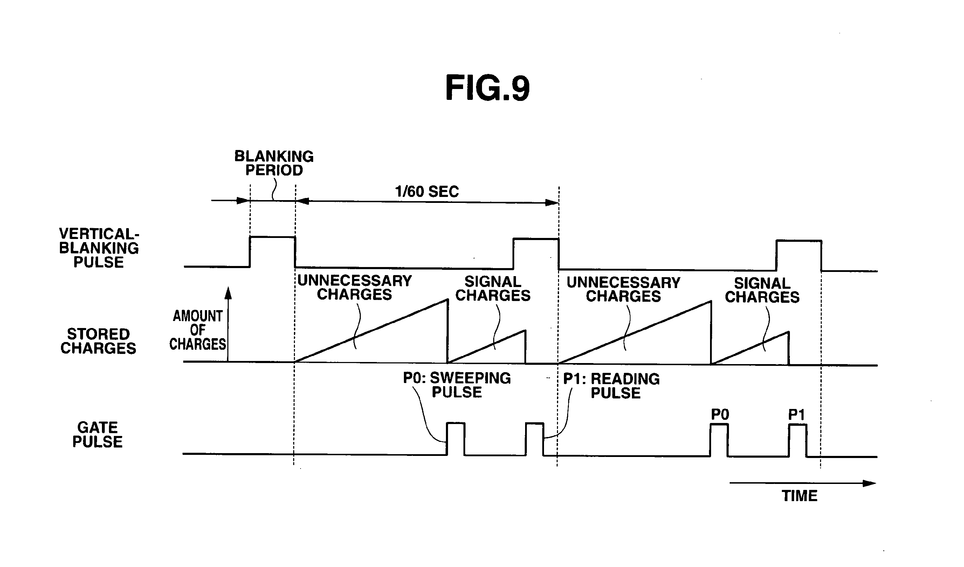

Fig. 9 is a diagram showing for explaining the principle of an electronic shutter further showing a relationship among a vertical blanking pulse, charges stored in the CCD, and the timing of gate pulse.

Referring to Fig. 9, the electronic shutter sweeps unnecessary charges stored in the CCD at the timing set by a sweeping pulse P0, and controls the time for storing the charges of signals read from a reading pulse P1. A period from the leading of the sweeping pulse P0 to the trailing of the reading pulse P1 indicates a charge storing time of signal (exposure time, that is, reciprocal of the speed of electronic shutter).

An electronic-shutter control signal is transmitted to the normal CCD driver 46 or the CCD driver 47 with high sensitivity, and is used for controlling the charge storing time of the CCD 27 or the CCD 28 with high sensitivity via the relay switch 29. For example, the scope used for observation with fluorescent light has the allowable diameter varied depending on the used part (down gastrointestinal-tract, up gastrointestinal-tract, and bronchi). Therefore, a scope with only one CCD can be used in addition to the scope with the two CCDs as shown in Fig. 1.

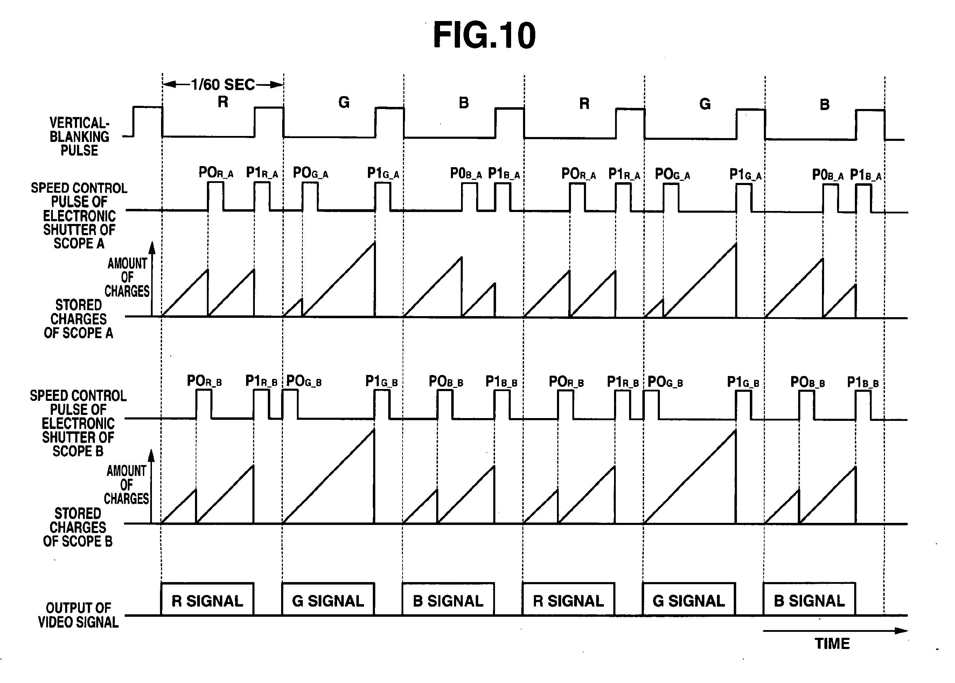

Since the scopes have the spectroscopy properties varied depending on objective optical systems 23 and 24 and the optical filters 25 and 26, the image brightness varies even with the same observation with fluorescent light. Therefore, the speed of electronic shutter is adjusted to correcting the brightness, depending on the type of scope 2. Incidentally, the speed of electronic shutter may be common to colors of the field-sequential light, or may be changed for colors as shown in Fig. 10.

Fig. 10 shows examples of two scopes A and B having the speed of electronic shutter varied depending on the colors. Reference symbols POR_A, POG_A, and POB_A denote sweeping pulses for R, G, and B colors of the scope A, reference symbols P1R A, P1G A, and P1B_ A denote reading pulses for R, G, and B colors of the scope A, reference symbols POR_B, POG_B, and P0B_B for R, G, and B colors denote sweeping pulses for R, G, and B colors of the scope B, and reference symbols PIR_B, PIG_B, and PIB_B denote reading pulses for R, G, and B colors of the scope B. The time interval between the sweeping pulse P0 and the reading pulse P1 in one period of the vertical blanking pulse determines the speed of electronic shutter. The sweeping pulse P0 and the reading pulse P1 function as speed control pulses of the electronic shutter.

The light-source control circuit 51 outputs a control signal based on the observing-mode identifying signal from the observing-mode switching circuit 45 so that the lamp driving circuit 7 of the light source 1, the motor 9, the motor 13, and the motor 14 are operated in accordance with the observing mode.

The light source 1 is operated based on the control signal outputted from the light-source control circuit 51 in the processor 3. The lamp driving circuit 7 has therein an element for storing the duty ratio of lamp driving current (not shown). The spectroscopy properties of the filters 21a, 21b, 21c, 22a, 22b, and 22c used for the rotary filter 12 are manufactured with the variation in the manufacturing step. Therefore, the light source 1 has the individual difference of the rotary filter 12, that is, the color balance has the individual difference. In order to correct the individual difference, the duty ratio of lamp driving current for changing the driving current of the lamp 8 by two steps is measured in advance at the manufacturing timing in the factory, and is stored in the element for storing duty ratio of lamp driving current.

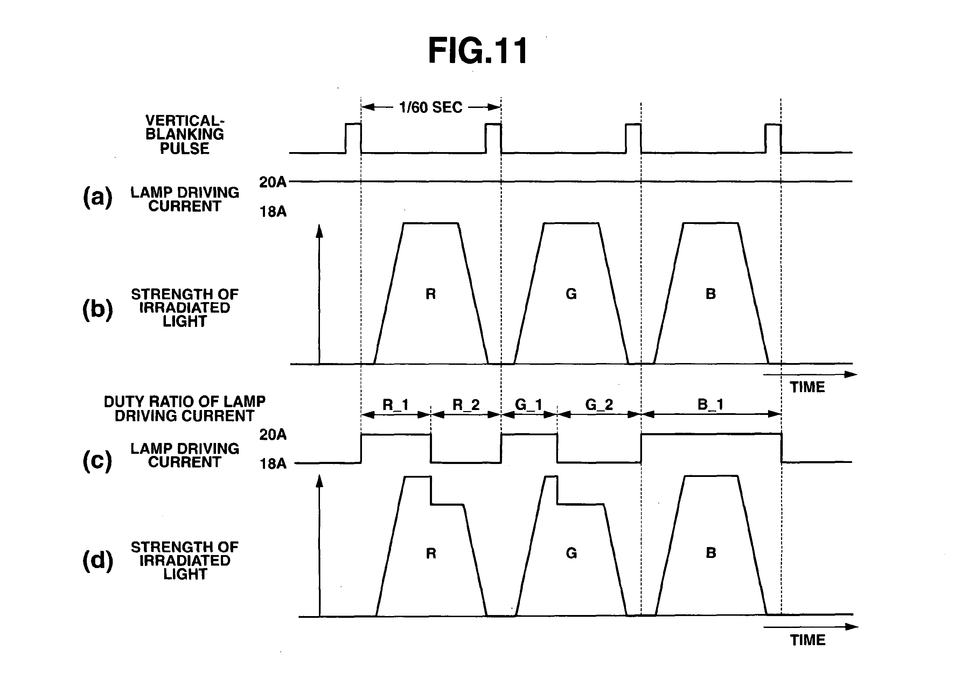

Fig. 11 is an explanatory diagram of the correction of the variation in color balances using the adjustment of the duty ratio of lamp driving current.

Without the duty ratio of lamp driving current, referring to Fig. 11(a), the driving current of the lamp 8 is constant and therefore the amount of irradiated light subjected to the field-sequential light using the rotary filter 12 is as shown in Fig. 11(b).

On the other hand, with the duty ratio of lamp driving current, rectangular waves are generated based on the duty ratio, as shown in Fig. 11(c). At the timing at which the irradiated light through the field-sequential processing is irradiated, the driving current of the lamp at the irradiating timings of colors is changed at two steps, thereby obtaining the amount of irradiated light as shown in Fig. 11(d).

Since the rotary filter 12 according to the first embodiment has a double structure, two types of the duty ratio of lamp driving current for inner circumference and outer circumference are stored in advance in the element for storing duty ratio of lamp driving current in the light source 1.

If the observing-mode identifying signal from the observing-mode switching circuit 45 is a signal indicating the observation with fluorescent light, the inner circumference of the rotary filter 12 is used. Therefore, an instruction is issued to the lamp driving circuit 7 so that the light-source control circuit 51 uses the duty ratio for inner circumference. If the observing-mode identifying signal from the observing-mode switching circuit 45 is a signal indicating the observation other than the observation with fluorescent light, the light-source control circuit 51 selects the duty ratio for outer circumference so as to use the outer circumference of the rotary filter 12. Thus, the amount of illuminating light is controlled and the individual difference of color balance of the light source 1 is corrected.

The filter turret 10 comprises the optical filters 17, 18, 19, and 20 having the spectroscopy properties varied depending on the observing mode. The motor 9 is rotationally driven by a control signal from the light-source control circuit 51 based on the observing-mode identifying signal so that the optical filter corresponding to the selected observing mode is moved on the optical path of the illuminating light. The motor 9 stops at a predetermined position, thereby fixing the filter turret 10.

The illuminating light passing through the optical filter of the filter turret 10 is adjusted with proper brightness by the illuminating-light stop 11. The adjusted light is converted into field-sequential light by the rotary filter 12 which is rotationally driven by the motor 13. The motor 13 has the rotating frequency varied depending on the observing mode, and is driven by the rotating frequency of 10 Hz in the observation with fluorescent light and is driven by the rotating frequency of 20 Hz in other observing modes.

If the observing-mode identifying signal indicates the observation with fluorescent light, the light-source control circuit 51 communicates data so that the rotating frequency of the rotary filter 12 is synchronized with the rotating frequency of 10 Hz. On the other hand, in the observing mode other than the observation with fluorescent light, the light-source control circuit 51 communicates data so that the rotating frequency of the rotary filter 12 is synchronized with the rotating frequency of 20 Hz.

In the observation with fluorescent light, the motor 14 is vertically driven based on the signal from the light-source control circuit 51 so that the inner circumference of the rotary filter 12 exists on the optical axis of the illuminating light. In the observing modes except for the observation with fluorescent light, similarly, the motor 14 is vertically driven based on the signal from the light-source control circuit 51 so that the outer circumference of the rotary filter 12 exists on the optical axis of the illuminating light.

The condenser lens 16 condenses the illuminating light passing through the rotary filter 12 on the incident surface of the light guide 15 in the scope 2 and the subject is irradiated with the condensed light. The return light is captured by the CCD 27 or the CCD 28 with high sensitivity.

Incidentally, according to the first embodiment, the endoscope using the field-sequential method is used. Further, the endoscope using the synchronous operating method may be used.

The scope 2 may be a fiber scope. In this case, a signal processing device may be detachable to an eyepiece unit of the fiber scope and may process an image signal captured by a solid-state image pickup device. The CCD 28 with high sensitivity is used only for the observation with fluorescent light and further may be used for another observing mode.

The scope 2 corresponding to the observation with specific light may have one CCD and the arranged CCD in this case may be the normal CCD or CCD with high sensitivity.

The install position of the observing-mode change-over switch 32 is not limited to the operating unit in the scope 2 and further may be a button arranged to the light source 1 or a font panel (not shown) of the processor 3, or a key (not shown) of a foot switch or keyboard connected to the processor 3.

Two or more observing-mode change-over switches 32 may be arranged. The electronic shutter may control the brightness in conjunction with the light control circuit 49.

Since the storage capacity of the scope-information storing element 31 is limited, the setting for each scope, of the priority or speed of electronic shutter stored in the memory (not shown) in the processor 3 may be read and be used based on information indicating two types of scopes 2 stored in the scope-information storing element 31.

[Advantage]

According to the first embodiment, as mentioned above, only the observing mode corresponding to the connected scope is switched in the order of higher priority. The scope does not select the observing mode without corresponding to the scope, thereby preventing the erroneous operation. Since it is possible to obtain the image subjected to the proper processing corresponding to the observing mode in accordance with the switching operation, the adjustment of setting using the manual operation is not necessary and the observing mode is easily switched.

[Second embodiment]

[Object]

It is an object to prevent the operation of a switch which does not effectively function depending on the observing mode.

[Structure]

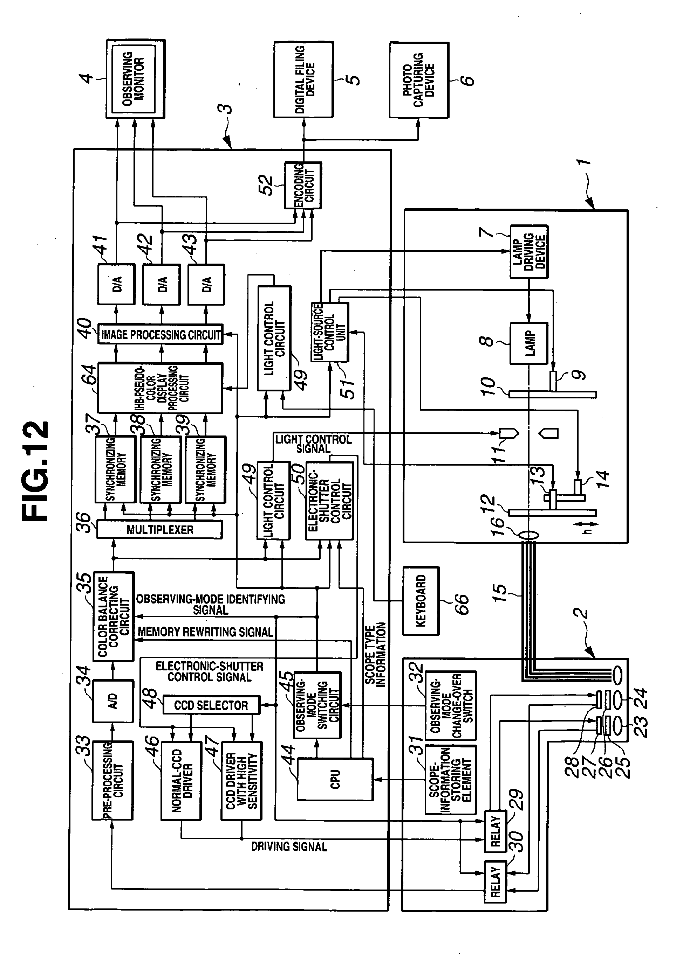

Fig. 12 is a diagram showing the entire structure of an endoscope device according to a second embodiment of the present invention.

The structure according to the second embodiment of the present invention is similar to that according to the first embodiment. Therefore, portions different from those according to the first embodiment are mainly described here.

The processor 3 according to the second embodiment comprises an IHb-pseudo-color display processing circuit 64 at the subsequent part of the synchronizing memories 37, 38, and 39. The image signal sequentially flows to the pre-processing circuit 33, the A/D converting circuit 34, the color balance correcting circuit 35, the multiplexer 36, the synchronizing memories 37, 38, and 39, the IHb-pseudo-color display processing circuit 64, the image processing circuit 40, and the D/A converting circuits 41, 42, and 43. Further, the processor 3 comprises: the CPU 44; the observing-mode switching circuit 45; the normal CCD driver 46, the CCD driver 47 with high sensitivity; the CCD selector 48; the light control circuit 49; the electronic-shutter control circuit 50; the light-source control circuit 51; the encoding circuit 52; and an IHb-pseudo-color processing control circuit 65.

A keyboard 66 is connected to the processor 3, and comprises an IHb-pseudo-color display switching key (not shown) for alternately switching the on/off operation of the IHb-pseudo-color display processing function.

[Operation]

The image signal outputted from the CCD 27 or the CCD 28 with high sensitivity which receives the return light from the subject is inputted to the IHb-pseudo-color display processing circuit 64, via the relay switch 30 and the pre-processing circuit 33, the A/D converting circuit 34, the color balance correcting circuit 35, the multiplexer 36, and the synchronizing memories 37, 38, and 39 in the processor 3.

As disclosed in Japanese Unexamined Patent Application Publication No. 2001-37718, the IHb-pseudo-color display processing circuit 64 calculates the value (hereinafter, abbreviated to an IHb) correlating with the hemoglobin content in the blood using the endoscope image in the observation with normal light, forms pseudo-color data, serving as pseudo-image data indicating the change of IHb, combines the formed data to the original endoscope image, and outputs the combined data. Since the change in IHb corresponds to the change of the volume of blood flow, the change in IHb can be used for the identification of the lesion or the normal part or the determination of the degree of inflammation.

Recently, a relationship between IHb and the helicobacter pylori (hereinafter, abbreviated to the HP) contributing to the cancer development has been researched and it has been suggested that the appearance of cancer can be diagnosed by referring to IHb. The IHb-pseudo-color display processing circuit 64 calculates a value defined by the following formula.

$$IHb = 32 \times Log_2(R/G) \qquad (1)$$

R:    data of R image
G:    data of G image

In the observation with specific light, the image signals obtained at the timing of R and G images of the rotary filter 12 become information different from that in the observation with normal light due to the difference in spectroscopy properties of the optical filters arranged to the rotary filter 12 or the filter turret 10 and further have calculated values based on Formula (1). Therefore, in the observation with specific light, the IHb-pseudo-color display processing circuit 64 does not calculate the data.

The observing-mode identifying signal outputted from the observing-mode switching circuit 45 is inputted to the IHb-pseudo-color processing control circuit 65. By pressing an IHb-pseudo-color display switching key (not shown) arranged to the keyboard 66, the switching signal to the IHb pseudo-color display operation is similarly inputted to the IHb-pseudo-color processing control circuit 65.

The IHb-pseudo-color processing control circuit 65 receives the switching signal to the IHb pseudo-color display operation. Only when the observing-mode identifying signal indicates the observation with normal light, the IHb-pseudo-color display processing circuit 64 outputs a signal indicating that the calculation based on Formula (1) is valid. When one of the two input signals does not exist, the IHb-pseudo-color display processing circuit 64 outputs a signal indicating that the IHb pseudo-color display processing is invalid.

The IHb-pseudo-color display processing circuit 64 receives the valid signal from the IHb-pseudo-color processing control circuit 65, then, calculates the image signals inputted from the synchronizing memories 37, 38, and 39 based on Formula (1), combines the pseudo-color data to the image signals, and outputs the combined signal to the image processing circuit 40. On the other hand, when the IHb-pseudo-color display processing circuit 64 receives the invalid signal, the signals are outputted to the image processing circuit 40 without processing the image signals inputted from the synchronizing memories 37, 38, and 39.

Therefore, in the observing mode with specific light, even by pressing the IHb-pseudo-color display switching key, the IHb-pseudo-color display processing circuit 64 receives the signal indicating that the processing is invalid, the processing is not performed and the image signals inputted from the synchronizing memories 37, 38, and 39 are outputted to the image processing circuit 40 without the processing. Incidentally, the IHb-pseudo-color display switching key switches the normal operation and the IHb pseudo-color display operation every key operation.

The processing 3 comprises warning means (not shown) which performs at least one of the generation of warning sound or warning display operation on the screen indicating that the IHb-pseudo-color display processing is invalid upon pressing the IHb-pseudo-color display switching key in the observing mode with specific light.

The sequential operation is similar to that according to the first embodiment.

Incidentally, according to the second embodiment, the endoscope using the field-sequential method is used. Further, the endoscope using the synchronous operating method is used.

The scope 2 may be a fiber scope. In this case, a signal processing device may be detachable to an eyepiece unit of the fiber scope and may process an image signal captured by a solid-state image pickup device. Only in the observation with fluorescent light, the CCD 28 with high sensitivity is used and further may be used another observing mode.

The scope 2 corresponding to the observation with specific light may have one CCD and the arranged CCD may be the normal CCD or the CCD with high sensitivity.

In the observation with specific light, the IHb-pseudo-color display processing is invalid. For example, as in the case of displaying the pseudo-color of the concentration of Indocyanine green (hereinafter, abbreviated to ICG) so as to observe the concentration of pigment, that is, ICG injected in the blood by the intravenous injection with the infrared light and to check the volume of blood flow or sentinel lymph node in the observing mode with infrared light, when the calculating result of Formula (1) has another advantage for diagnosis, the function of the IHb-pseudo-color display processing circuit 64 may be valid even in the observing mode.

A circuit for changing whether or not the processing is valid in accordance with the observing mode is not limited to the IHb-pseudo-color display processing circuit 64.

The switching to the IHb-pseudo-color display operation is not limited to the key on the keyboard, and may be a button arranged to the light source 1 or a font panel (not shown) of the light source 1 or the processor 3, or a key (not shown) of a foot switch or a switch arranged to the operating unit of the scope.

Two or more observing-mode change-over switches 32 may be arranged. Further, the warning means may indicate the warning state with the light-on operation of light-emitting means, such as an LED, as well as the generation of warning sound and the display operation on the screen.

In order to prevent the erroneous operation, means for displaying a function used for the screen or erroneous-operation preventing means for warning by an available change-over switch with the light-on operation of LED may be added in advance.

Since the storage capacity of the observing-mode change-over switch 32 is limited, the setting for each scope, stored in the memory (not shown) in the processor 3 may be read and be used based on the information indicating two types of scopes 2 stored in the scope-information storing element 31.

[Advantage]

According to the second embodiment, as mentioned above, it is possible to prevent the operation of the switch which does not effectively function depending on the observing mode.

[Third embodiment]

[Object]

Upon selecting the observing mode without corresponding to the scope, the operation of the endoscope device is prevented.

[Structure]

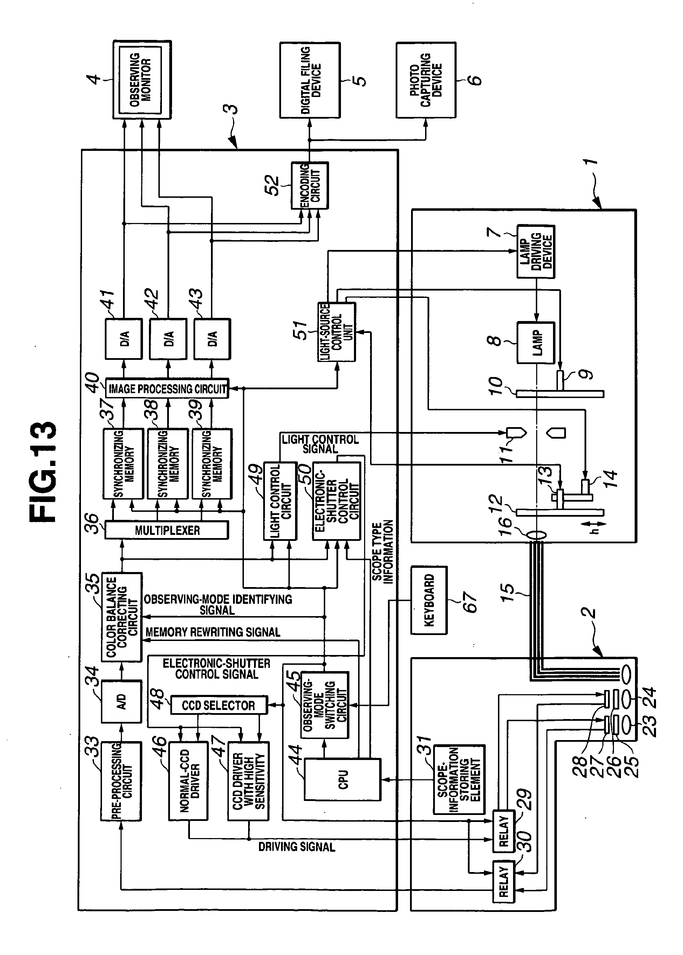

Fig. 13 is a diagram showing the entire structure of an endoscope device according to a third embodiment of the present invention.

The structure according to the third embodiment of the present invention is similar to those according to the first and second embodiments. Therefore, portions different from those according to the first and second embodiments are mainly described here.

In the processor 3 according to the third embodiment, the image signal sequentially flows to the pre-processing circuit 33, the A/D converting circuit 34, the color balance correcting circuit 35, the multiplexer 36, the synchronizing memories 37, 38, and 39, the image processing circuit 40, and the D/A converting circuits 41, 42, and 43. The processor 3 comprises: the CPU 44; the observing-mode switching circuit 45; the normal CCD driver 46; the CCD driver 47 with high sensitivity; the CCD selector 48; the light control circuit 49; the electronic-shutter control circuit 50; the light-source control circuit 51; and the encoding circuit 52.

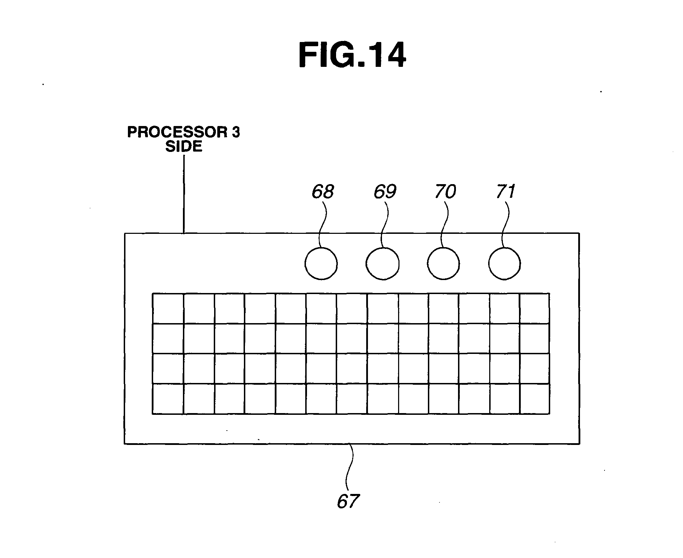

Referring to Fig. 14, a keyboard 67 is connected to the processor 3, and comprises observing-mode switching keys 68, 69, 70, and 71 which switch the observing mode to the observation with normal light, the observation with fluorescent light, the observation with infrared light, and the observation with narrow-band light.

[Operation]

Similarly to the first embodiment, the power of the scope 2 is turned on while the scope 2 is connected to the light source 1 and the processor 3, the endoscope device starts in the observing mode with normal light. Simultaneously with the start operation, the scope-information storing element 31 in the scope 2 reads the information on the observing mode corresponding to the scope 2 to the CPU 44 in the processor 3 and stores the information.

The keyboard 67 is connected to the processor 3, thereby inputting patient information or necessary comment in the examination. The keyboard 67 comprises keys for inputting alphabet and keys for inputting numbers. Further, the keyboard 67 has a space for arranging the keys and therefore has a number of observing-mode switching keys corresponding to that of observing modes of the processor 3. By pressing the observing mode which is used by a user, the observing mode is directly switched to the selected observing mode, without considering the priority of the observing mode described according to the first embodiment.

By pressing any of the observing-mode switching keys 68, 69, 70, and 71, the observing-mode switching signal is inputted from the keyboard 67 to the observing-mode switching circuit 45 in the processor 3. The observing-mode switching circuit 45 receives the signal indicating the type of observing mode corresponding to the scope 2 from the CPU 44. The received signal is stored in a memory (not shown) arranged in the observing-mode switching circuit 45.

In the observing-mode switching circuit 45, the observing-mode switching signal is compared with the type of observing modes stored in the memory in the observing-mode switching circuit 45. When the observing-mode switching signal matches the type of observing modes stored in the memory, an observing-mode switching signal for instructing the switching to the selected observing mode is outputted.

On the other hand, when the observing-mode switching signal does not match the type of observing modes stored in the memory, the observing-mode switching signal is not outputted and the current observing mode keeps. Warning means (not shown) indicates, to the user, that the connected scope 2 does not correspond to the observing mode selected by the observing-mode switching keys in the keyboard 67 with at least one of the generation of warning sound, warning display operation on the screen, or light-on operation of light-emitting means, such as an LED.

When the observing mode selected by the observing-mode switching keys of the keyboard 67 corresponds to scope 2, the observing-mode identifying signal outputted from the observing-mode switching circuit 45 is transmitted to the CCD selector 48, the color balance correcting circuit 35, the synchronizing memories 37, 38, and 39, the image processing circuit 40, the light control circuit 49, the electronic-shutter control circuit 50, and the light-source control circuit 51 in the processor 3 and the relay switches 29 and 30 in the scope 2.

The subsequent operations are the same as those according to the first embodiment.

Incidentally, according to the third embodiment, the endoscope using the field-sequential method is used. Further, the endoscope using the synchronous operating method may be used. The scope 2 may be a fiber scope. In this case, a signal processing device may be detachable to an eyepiece unit of the fiber scope and may process an image signal captured by a solid-state image pickup device.

The scope 2 corresponding to the observation with specific light may have one CCD and the arranged CCD may be the normal CCD or the CCD with high sensitivity.

The switching operation using the keyboard operation according to the third embodiment can be used commonly with the operation for sequentially switching the observing modes in accordance with the priority of the observing mode of the scope 2 with a single change-over switch according to the first embodiment.

The observing-mode switching keys 68, 69, 70, and 71 are arranged to the keyboard 67 in consideration of the relationship of the install space. If the install space has a sufficient margin, the install space may be the operating unit in the scope 2, a button on a front panel (not shown) of the light source 1 or the processor 3, or a button of a footswitch or a remote controller.

In addition to the warning means, it is possible to add erroneous-operation preventing means, such as a function for displaying the observing mode corresponding to the scope 2 on the observing monitor 4 or a function for lighting-on light-emitting means, such as an LED, arranged to the key, only for the switching key to the observing mode corresponding to the scope 2 among the observing-mode switching keys 68, 69, 70, and 71.

Since the storage capacity of the scope-information storing element 31 is limited, the setting for each scope, of the priority or speed of electronic shutter stored in the memory (not shown) in the processor 3, may be read and be used based on information indicating two types of scopes 2 stored in the scope-information storing element 31.

[Advantage]

According to the third embodiment, as mentioned above, it is possible to prevent the selection of the observing mode without corresponding to the scope.

The present invention is not limited to the above-mentioned embodiments and can be variously modified without departing the essentials of the present invention.

Incidentally, the above-mentioned embodiments are partly combined and the combined embodiment belongs to the present invention.

The present invention is not limited to the above-mentioned embodiments and can be variously modified without departing the essentials of the present invention.

Industrial Applicability

According to the present invention, in the endoscope device corresponding to a plurality of observing modes, e.g., the observation with normal light and at least one observation with specific light, only the observing mode corresponding to the connected scope is selected and is executed, thereby preventing the erroneous operation. Further, the setting is not necessary every examination.

Cross-Reference to Related Application

The present invention is applied based on the priority of Japanese Examined Patent Application Publication No. 2003-175427 in Japan on 19, June, 2003. The disclosure is referred to the specification, claims, and drawings of the present invention.

**Claims**

1. An endoscope device comprising:

   an endoscope comprising an image pickup device for capturing an image of a subject, the endoscope that can observe the subject in a predetermined observing mode; and
   a signal processing device comprising an identifying unit that identifies the observing mode of a connected endoscope based on information from the connected endoscope, the signal processing device that receives a signal from the image pickup device, can execute signal processing corresponding to a plurality of observing modes including the predetermined observing mode, and further can execute only signal processing corresponding to the observing mode identified by the identifying unit.

2. An endoscope device comprising:

   an endoscope that can observe a subject in a predetermined observing mode;
   an image pickup device that is detachable to an eyepiece unit in the endoscope and captures an image of the subject; and
   a signal processing device comprising an identifying unit that identifies the observing mode of a connected endoscope based on information from the connected endoscope, the signal processing device that receives a signal from the image pickup device, can execute signal processing corresponding to a plurality of observing modes including the predetermined observing mode, and further can execute only signal processing corresponding to the observing mode identified by the identifying unit.

3. A signal processing device comprising an image pickup device that captures an image of a subject, the signal processing device being connected to an endoscope that can observe the subject in a predetermined observing mode and processing a signal from the image pickup device in the endoscope, wherein the signal processing device comprises an identifying unit that identifies the observing mode of a connected endoscope based on information from the connected endoscope, and receives a signal from the image pickup device, can execute signal processing corresponding to a plurality of observing modes including the predetermined observing mode, and further can execute only signal processing corresponding to the observing mode identified by the identifying unit.

4. An endoscope device comprising:

   an endoscope corresponding to an observing mode with normal light and at least one observing mode with specific light;
   a solid-state image pickup device that is arranged to the distal end of the endoscope and receives light of the subject image;
   a signal processing device that performs signal processing varied depending on the observing mode of the endoscope;
   storing means that stores observing-mode information of the endoscope; and
   observing-mode switching means that switches the observing mode based on information stored in the storing means.

5. The endoscope device according to Claim 4, wherein the storing means stores the observing-mode information of the endoscope and the priority of observing mode in the switching operation of the observing mode.

6. The endoscope device according to Claim 4, wherein the storing means is a storing element that is arranged to the endoscope.

7. The endoscope device according to Claim 4, wherein the storing means comprises a storing element that is arranged to the endoscope and a storing unit that is arranged in the signal processing device.

8. The endoscope device according to Claim 4, wherein the observing mode with specific light includes at least one of observation with fluorescent light, observation with infrared light, and observation with narrow-band light.

9. An endoscope device comprising
   an endoscope corresponding to an observing mode with normal light and at least one observing mode with specific light;
   an image pickup device that is detachable to an eyepiece unit of the endoscope and comprises a solid-state image pickup device;
   a signal processing device that performs signal processing varied depending on the observing mode of the endoscope;
   storing means that stores observing-mode information of the endoscope; and
   observing-mode switching means that switches the observing mode based on the information stored in the storing means.

10. The endoscope device according to Claim 9, wherein the storing means stores the observing-mode information corresponding to the endoscope and the priority of the observing mode in the switching operation of the observing mode.

11. The endoscope device according to Claim 9, wherein the storing means is a storing element that is arranged to the endoscope.

12. The endoscope device according to Claim 9, wherein the storing means comprises a storing element that is arranged to the endoscope and a storing unit that is arranged in the signal processing device.

13. The endoscope device according to Claim 9, wherein the observing mode with specific light includes at least one of observation with fluorescent light, observation with infrared light, and observation with narrow-band light.

# FIG.1

# FIG.2

# FIG.3

# FIG.4

# FIG.5

WAVELENGTH

# FIG.6

EP 1 637 062 A1

# FIG.7

*35*

MEMORY REWRITING
SIGNAL FROM CPU 44

*57a*

CORRECTING-
COEFFICIENT
STORING
MEMORY

*58*

*57b*

CORRECTING-
COEFFICIENT
STORING
MEMORY

SELECTOR

OBSERVING-MODE
IDENTIFYING SIGNAL

*57c*

CORRECTING-
COEFFICIENT
STORING
MEMORY

INPUT IMAGE SIGNAL

OUTPUT IMAGE
SIGNAL

×

*59*

# FIG.8

OBSERVING-MODE
IDENTIFYING SIGNAL

| OBSERVING MODE | ID SIGNAL |
|---|---|
| NORMAL LIGHT | 00 |
| FLUORESCENT LIGHT | 01 |
| INFRARED LIGHT | 10 |
| NARROW-BAND LIGHT | 11 |

60

61

| ID SIGNAL | ADDRESS |
|---|---|
| 00 | 0010 |
| 01 | 0020 |
| 10 | 0030 |
| 11 | 0040 |

62

| ADDRESS | FILTER COEFFICIENT |
|---|---|
| 0010 | A |
| 0020 | A |
| 0030 | A |
| 0040 | A |

63

SPATIAL FILTER PROCESSING CIRCUIT

IMAGE SIGNAL AFTER PROCESSING

IMAGE SIGNAL

EP 1 637 062 A1

# FIG.9

BLANKING PERIOD

1/60 SEC

VERTICAL-BLANKING PULSE

STORED CHARGES

AMOUNT OF CHARGES

UNNECESSARY CHARGES

SIGNAL CHARGES

UNNECESSARY CHARGES

SIGNAL CHARGES

GATE PULSE

P0: SWEEPING PULSE

P1: READING PULSE

P0

P1

TIME

# FIG.10

# FIG.11

VERTICAL-
BLANKING
PULSE

$\longleftarrow$ 1/60 SEC $\longrightarrow$

(a) LAMP DRIVING CURRENT

20A

18A

(b) STRENGTH OF IRRADIATED LIGHT

R     G     B

TIME

DUTY RATIO OF LAMP DRIVING CURRENT

R_1   R_2   G_1   G_2   B_1

(c) LAMP DRIVING CURRENT

20A

18A

(d) STRENGTH OF IRRADIATED LIGHT

R     G     B

TIME

EP 1 637 062 A1

# FIG.12

EP 1 637 062 A1

# FIG.13

EP 1 637 062 A1

# FIG.14

PROCESSOR 3
SIDE

*68*    *69*    *70*    *71*

*67*

## INTERNATIONAL SEARCH REPORT

| International application No. |
| --- |
| PCT/JP2004/008918 |

**A. CLASSIFICATION OF SUBJECT MATTER**
Int.Cl⁷ A61B1/00

According to International Patent Classification (IPC) or to both national classification and IPC

**B. FIELDS SEARCHED**

Minimum documentation searched (classification system followed by classification symbols)
Int.Cl⁷ A61B1/00-1/32

Documentation searched other than minimum documentation to the extent that such documents are included in the fields searched

| | | | |
| --- | --- | --- | --- |
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br><br>A | JP 2003-126015 A (Olympus Optical Co., Ltd.),<br>07 May, 2003 (07.05.03),<br>Full text; Figs. 1 to 12<br>Full text; Figs. 1 to 12<br><br>Full text; Figs. 1 to 12<br>& EP 1258221 A2 & US 2002/0175993 A1 | 1,3<br>2,4,6,8,9,<br>11,13<br>5,7,10,12 |
| X<br>Y<br><br>A | JP 2002-126015 A (Olympus Optical Co., Ltd.),<br>26 November, 2002 (26.11.02),<br>Full text; Figs. 1 to 24<br>Full text; Figs. 1 to 24<br><br>Full text; Figs. 1 to 24<br>& EP 1258220 A2 & US 2002/0177751 A1 | 1,3<br>2,4,6,8,9,<br>11,13<br>5,7,10,12 |

☒ Further documents are listed in the continuation of Box C.  ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>06 July, 2004 (06.07.04) | Date of mailing of the international search report<br>20 July, 2004 (20.07.04) |
| --- | --- |
| Name and mailing address of the ISA/<br>    Japanese Patent Office | Authorized officer |
| Facsimile No. | Telephone No. |

Form PCT/ISA/210 (second sheet) (January 2004)

**INTERNATIONAL SEARCH REPORT**

| International application No. |
| --- |
| PCT/JP2004/008918 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2000-354583 A (Olympus Optical Co., Ltd.), 26 December, 2000 (26.12.00), Full text; Figs. 1 to 7 (Family: none) | 2 |
| Y | JP 2001-29313 A (Olympus Optical Co., Ltd.), 06 February, 2001 (06.02.01), Full text; Figs. 1 to 29 & WO 00/69324 A1 & EP 1099405 A1 | 4,6,8,9,11, 13 |

Form PCT/ISA/210 (continuation of second sheet) (January 2004)